# EUROPEAN PATENT APPLICATION

(11) **EP 2 684 892 A1**
(43) Date of publication of application: **15.01.2014**
(21) Application number: 12305855.4
(22) Date of filing: 13.07.2012
(51) Int. Cl.: C07K 14/47, C12N 9/22, C12N 15/86, C12N 15/90, A61K 48/00

(54) **Compositions and methods for duchenne muscular dystrophy gene therapy**

(71) Applicant: Association Française contre les Myopathies, 75651 Paris Cedex 13 (FR)
(72) Inventor: Popplewell, Linda Jane, Ascot, Berkshire SL5 0AA (GB); Dickson, John George, London, NW7 4NR (GB); Yáñez-Muñoz, Rafael Joaquín, Bromley, BR1 2HA (GB)
(74) Representative: Sekhri, Redha

(57) **Abstract**

The present invention relates to a gene therapy method for the treatment of Duchenne muscular dystrophy, or DMD.

SEQUENCE LISTING

<110>  ASSOCIATION FRANCAISE CONTRE LES MYOPATHIES

<120>  COMPOSITIONS AND METHODS FOR DUCHENNE MUSCULAR DYSTROPHY GENE
       THERAPY

<130>  B1364EP00

<160>  26

<170>  PatentIn version 3.5

<210>  1
<211>  24
<212>  DNA
<213>  artificial

<220>
<223>  MN-DMD31 intron 44 target site

<400>  1
aatgtctgat gttcaatgtg ttga                                              24


<210>  2
<211>  1000
<212>  DNA
<213>  artificial

<220>
<223>  left arm of homology 1 kb

<400>  2
aaccattttt ttaaaaaaaa tggaactcca attgtacatt tattaggctc cttaaagtgc      60

cccactactc actgatgtta tgttcattgt ctgtttggtc tctcttttct ctgtaatttg     120

ttttatataa tctctattgt caaattgact aatctttttc aaagtctaat ctatggctaa     180

tcccatgtag tatatatttt taacatcaga cattttcatc tcttagaagt aaaagttggg     240

tctttttatt tcttccatgt gtctactcaa catgttcagt ctttactttc ttgactatat     300

ggaatacaga tataataact gttagaatat tcttctctac taattttatc atctgtgtct     360

attctgggtt aatttaaatt gatttatttt tctcctcatt aagtgtgttg tttaactgct     420

tctttggatg actggtaatt tttgactata tgccagacat tgtgaatttt aacttagcgc     480

atgcttgata cttcaaataa attcaaatat attgaaataa atattctcaa ccctcgttct     540

ggaacacagt taattcactt ggaaacaatt tgatcttttg agaatcttcc ttttatgctt     600

tgttatgacc agaacagtgt aagtttaggg ctactttttc cccactactg aggcaaaacc     660

cttctgagta ctctctctga tgtcctgtga atgataaaat ttttcactgg ggctcgtggg     720

aacaggtggt attactagcc acgtgtgagc tctggtgatt gtttccttta attcttttgt     780

gaagttcttt ccttagcttt gagtggtttt cttgcataca tgaactgatc aagactcaga     840

tgaagaataa aataaagctt tctacaaatc tccaaaattt cctctgtgta tatatcacct     900

ctctggtatt ttgccctgtg atcactagtc agccttgggc tgctgaaact ctcagcttca     960

tcttttaaca gaagcctcct ggcaaggatc actgtccttc 1000

<210> 3
<211> 1500
<212> DNA
<213> artificial

<220>
<223> left arm of homology 1.5 kb

<400> 3
tgctttaata tagaagatac attttaaaaa tagcactgaa gtgaattttg ggctaatgta 60

gcataatggg gtttctgcct gagaggcaga aacatattag agttatataa aatgttttgg 120

ggtagatata gaaaccactt gccattttca atgatatcca acccaaggta gttatatatt 180

tcaatttata ttttattatc aaattagtac ttattgtgaa aaaaatcaag taacatagaa 240

atttgtaaaa gtacctccat tctactcttt ggaggatagt tgttcagtat gaattttgct 300

acatatttca ggctgggttt cttggaaagc cattgtaaaa tggagatttg tatgtagaag 360

gttaactagg gagtactttt acgatgaagc aatttgtttt gatgtaactt ggtgtagttt 420

tcttcatgtt tcttgttctt gaagtcagtt aagctcttga atctgtgcat ttaacatttc 480

atcaaattta gaaacctttc aaccattttt ttaaaaaaaa tggaactcca attgtacatt 540

tattaggctc cttaaagtgc cccactactc actgatgtta tgttcattgt ctgtttggtc 600

tctcttttct ctgtaatttg ttttatataa tctctattgt caaattgact aatctttttc 660

aaagtctaat ctatggctaa tcccatgtag tatatatttt taacatcaga cattttcatc 720

tcttagaagt aaaagttggg tctttttatt tcttccatgt gtctactcaa catgttcagt 780

ctttactttc ttgactatat ggaatacaga tataataact gttagaatat tcttctctac 840

taattttatc atctgtgtct attctgggtt aatttaaatt gatttatttt tctcctcatt 900

aagtgtgttg tttaactgct tctttggatg actggtaatt tttgactata tgccagacat 960

tgtgaatttt aacttagcgc atgcttgata cttcaaataa attcaaatat attgaaataa 1020

atattctcaa ccctcgttct ggaacacagt taattcactt ggaaacaatt tgatcttttg 1080

agaatcttcc ttttatgctt tgttatgacc agaacagtgt aagtttaggg ctactttttc 1140

cccactactg aggcaaaacc cttctgagta ctctctctga tgtcctgtga atgataaaat 1200

ttttcactgg ggctcgtggg aacaggtggt attactagcc acgtgtgagc tctggtgatt 1260

gtttccttta attcttttgt gaagttcttt ccttagcttt gagtggtttt cttgcataca 1320

tgaactgatc aagactcaga tgaagaataa aataaagctt tctacaaatc tccaaaattt 1380

cctctgtgta tatatcacct ctctggtatt ttgccctgtg atcactagtc agccttgggc 1440

tgctgaaact ctcagcttca tcttttaaca gaagcctcct ggcaaggatc actgtccttc 1500

<210> 4

<211>  952
<212>  DNA
<213>  artificial

<220>
<223>  right arm of homology 1 kb

<400>  4
aagtgtttca ggtgtttcag gcaggagatt aagttcagcc tcctttactc caacttgaaa        60

acaagtccaa aacaaactat tttgatgtaa tttgatcttt taatacatta acattacaca       120

attttgtgaa tatatcataa tttaaaattt tcagagaatg tctaatggtc ctcatttctt       180

gacagtgtgg tttagttgaa actgatgaac attttatcaa aacttttccc ctcaattgga       240

tacttttttt tttttgagat ggaattttgc ttttgtcacc caggctggag tggcatgatc       300

tcagctcact gcaacctctg cctccaggct tcaagcaatt ctcctgcctt agcctcccga       360

gtagctggga ttacaggtgc ccacccccac acctggctaa tttttgtatt tttagtagag       420

acgagatttc accatgttgg tcaggctggt ctagatctcc gacctcaggt ggtctgcctg       480

tctcagcctc ccaaagtgct gggattgcag acgtgagcca ccatgcctgg ccaactggat       540

aattttaaaa agaccatttt atttagtcta ttttttctca atctatagat gagataagaa       600

aaatcattct agatgtccaa ggaaaaattc tttcagaaaa gagctgtgaa tgatatcaca       660

aaccccccaa acagttaagg tatttctttc ctggttattt tatgtccaaa atcatgcata       720

tgaacatgtg cacacacatg agcgtgcaca cacacatgaa tacatataca cgcacataat       780

gtaccttagg ttatctttcc attctgagta attatcgtaa aatgggtaaa atcaaccccg       840

taagatacct tcatcgataa ggcaaatcaa agctttggta atttctgcta tcttggcctt       900

tgttgattga ctaataatga ataagagaat gagtttcaat atttactatg aa               952


<210>  5
<211>  1500
<212>  DNA
<213>  artificial

<220>
<223>  right arm of homology 1.5 kb

<400>  5
aagtgtttca ggtgtttcag gcaggagatt aagttcagcc tcctttactc caacttgaaa        60

acaagtccaa aacaaactat tttgatgtaa tttgatcttt taatacatta acattacaca       120

attttgtgaa tatatcataa tttaaaattt tcagagaatg tctaatggtc ctcatttctt       180

gacagtgtgg tttagttgaa actgatgaac attttatcaa aacttttccc ctcaattgga       240

tacttttttt tttttgagat ggaattttgc ttttgtcacc caggctggag tggcatgatc       300

tcagctcact gcaacctctg cctccaggct tcaagcaatt ctcctgcctt agcctcccga       360

gtagctggga ttacaggtgc ccacccccac acctggctaa tttttgtatt tttagtagag       420

acgagatttc accatgttgg tcaggctggt ctagatctcc gacctcaggt ggtctgcctg       480

tctcagcctc ccaaagtgct gggattgcag acgtgagcca ccatgcctgg ccaactggat 540

aattttaaaa agaccatttt atttagtcta ttttttctca atctatagat gagataagaa 600

aaatcattct agatgtccaa ggaaaaattc tttcagaaaa gagctgtgaa tgatatcaca 660

aaccccccaa acagttaagg tatttctttc ctggttattt tatgtccaaa atcatgcata 720

tgaacatgtg cacacacatg agcgtgcaca cacacatgaa tacatataca cgcacataat 780

gtaccttagg ttatctttcc attctgagta attatcgtaa aatgggtaaa atcaaccccg 840

taagatacct tcatcgataa ggcaaatcaa agctttggta atttctgcta tcttggcctt 900

tgttgattga ctaataatga ataagagaat gagtttcaat atttactatg aaattatttt 960

agaagacagg atgtagacag tggctgttag caggcaattg tttggcatga gccagtaatg 1020

gttactgtga aaaaaatcaa ccaagcagcc catatattaa acaaacacac gcagaagcac 1080

gttggagtct gaagcctcat atgtacaatt ttcagtaaag aaataacttt tagatatgaa 1140

ataaacaaat agatatatgt tgtaaacttg tccctatgta ttttgatcaa attgcatcat 1200

atttttttca ctttaaagaa gagaatttag tgctttaact gagacttagt gttatcattc 1260

aaaatatact gactgccaat agcagcagaa agataatctg gttccatgca actctatttt 1320

ttttcctctg tcgcaagtaa aagacaaaat taagtacatg aattagtgct ttttgaagat 1380

attccagagc aatataccat gccactatgg agaacctctc taaaaatatc ccattttttt 1440

acctgagaaa aatattgatc atgttatatg ccactcaaat tggtttatta aattcgttga 1500

<210> 6
<211> 83
<212> DNA
<213> artificial

<220>
<223> synthetic splice acceptor

<400> 6
cttgtcgaga cagagaagac tcttgcgttt ctgataggca cctattggtc ttactgacat 60

ccactttgcc tttctctcca cag 83

<210> 7
<211> 74
<212> DNA
<213> artificial

<220>
<223> synthetic splice donor

<220>
<221> misc_feature
<222> (56)..(74)
<223> DISE element

<400> 7

gtaagtatca aggttacaag acaggtttaa ggagaccaat agaaactggg ctctttcttt 60

ccatgggttg gcct 74

<210> 8
<211> 372
<212> DNA
<213> artificial

<220>
<223> transcriptional terminator (bovine growth hormone polyA)

<400> 8
agatttcgat tccaccgccg ccttctatga aaggttgggc ttcggaatcg ttttccggga 60

cgccggctgg atgatcctcc agcgcgggga tctcatgctg gagttcttcg cccaccccaa 120

cttgtttatt gcagcttata atggttacaa ataaagcaat agcatcacaa atttcacaaa 180

taaagcattt ttttcactgc attctagttg tggtttgtcc aaactcatca atgtatctta 240

tcatgtctgt ataccgtcga cctctagcta gagcttggcg taatcatggt catagctgtt 300

tcctgtgtga aattgttatc cgctcacaat tccacacaac atacgagccg gaagcataaa 360

gtgtaaagcc tg 372

<210> 9
<211> 590
<212> DNA
<213> artificial

<220>
<223> Shortened Woodchuck Post-transcriptional Regulatory Element
      (WPRE)

<400> 9
taatcaacct ctggattaca aaatttgtga aagattgact ggtattctta actatgttgc 60

tccttttacg ctatgtggat acgctgcttt aatgcctttg tatcatgcta ttgcttcccg 120

tatggctttc attttctcct ccttgtataa atcctggttg ctgtctcttt atgaggagtt 180

gtggcccgtt gtcaggcaac gtggcgtggt gtgcactgtg tttgctgacg caacccccac 240

tggttggggc attgccacca cctgtcagct cctttccggg actttcgctt tccccctccc 300

tattgccacg gcggaactca tcgccgcctg ccttgcccgc tgctggacag gggctcggct 360

gttgggcact gacaattccg tggtgttgtc ggggaaatca tcgtcctttc cttggctgct 420

cgcctgtgtt gccacctgga ttctgcgcgg gacgtccttc tgctacgtcc cttcggccct 480

caatccagcg gaccttcctt cccgcggcct gctgccggct ctgcggcctc ttccgcgtct 540

tcgccttcgc cctcagacga gtcggatctc cctttgggcc gcctccccgc 590

<210> 10
<211> 1222
<212> DNA
<213> artificial

<220>
<223>  cDNA encoding DMD exons 45 to 52 (with silent mutations in
       positions 6 and 1539)

<400>  10
gaactgcagg atggcattgg gcagcggcaa actgttgtca gaacattgaa tgcaactggg      60

gaagaaataa ttcagcaatc ctcaaaaaca gatgccagta ttctacagga aaaattggga     120

agcctgaatc tgcggtggca ggaggtctgc aaacagctgt cagacagaaa aaagaggcta     180

gaagaacaaa agaatatctt gtcagaattt caaagagatt taaatgaatt tgttttatgg     240

ttggaggaag cagataacat tgctagtatc ccacttgaac ctggaaaaga gcagcaacta     300

aaagaaaagc ttgagcaagt caagttactg gtggaagagt tgcccctgcg ccagggaatt     360

ctcaaacaat taaatgaaac tggaggaccc gtgcttgtaa gtgctcccat aagcccagaa     420

gagcaagata aacttgaaaa taagctcaag cagacaaatc tccagtggat aaaggtttcc     480

agagctttac ctgagaaaca aggagaaatt gaagctcaaa taaaagacct tgggcagctt     540

gaaaaaaagc ttgaagacct tgaagagcag ttaaatcatc tgctgctgtg gttatctcct     600

attaggaatc agttggaaat ttataaccaa ccaaaccaag aaggaccatt tgacgttaag     660

gaaactgaaa tagcagttca agctaaacaa ccggatgtgg aagagatttt gtctaaaggg     720

cagcatttgt acaaggaaaa accagccact cagccagtga agaggaagtt agaagatctg     780

agctctgagt ggaaggcggt aaaccgttta cttcaagagc tgagggcaaa gcagcctgac     840

ctagctcctg gactgaccac tattggagcc tctcctactc agactgttac tctggtgaca     900

caacctgtgg ttactaagga aactgccatc tccaaactag aaatgccatc ttccttgatg     960

ttggaggtac ctgctctggc agatttcaac cgggcttgga cagaacttac cgactggctt    1020

tctctgcttg atcaagttat aaaatcacag agggtgatgg tgggtgacct tgaggatatc    1080

aacgagatga tcatcaagca gaaggcaaca atgcaggatt tggaacagag gcgtccccag    1140

ttggaagaac tcattaccgc tgcccaaaat ttgaaaaaca agaccagcaa tcaagaggct    1200

agaacaatca ttacggaccg aa                                             1222

<210>  11
<211>  4620
<212>  DNA
<213>  artificial

<220>
<223>  cDNA encoding DMD exons 45 to 79 (with silent mutation in
       position 6)

<400>  11
gaactgcagg atggcattgg gcagcggcaa actgttgtca gaacattgaa tgcaactggg      60

gaagaaataa ttcagcaatc ctcaaaaaca gatgccagta ttctacagga aaaattggga     120

agcctgaatc tgcggtggca ggaggtctgc aaacagctgt cagacagaaa aaagaggcta     180

gaagaacaaa agaatatctt gtcagaattt caaagagatt taaatgaatt tgttttatgg     240

ttggaggaag cagataacat tgctagtatc ccacttgaac ctggaaaaga gcagcaacta 300
aaagaaaagc ttgagcaagt caagttactg gtggaagagt tgcccctgcg ccagggaatt 360
ctcaaacaat taaatgaaac tggaggaccc gtgcttgtaa gtgctcccat aagcccagaa 420
gagcaagata aacttgaaaa taagctcaag cagacaaatc tccagtggat aaaggtttcc 480
agagctttac ctgagaaaca aggagaaatt gaagctcaaa taaaagacct tgggcagctt 540
gaaaaaaagc ttgaagacct tgaagagcag ttaaatcatc tgctgctgtg gttatctcct 600
attaggaatc agttggaaat ttataaccaa ccaaaccaag aaggaccatt tgacgttaag 660
gaaactgaaa tagcagttca agctaaacaa ccggatgtgg aagagatttt gtctaaaggg 720
cagcatttgt acaaggaaaa accagccact cagccagtga agaggaagtt agaagatctg 780
agctctgagt ggaaggcggt aaaccgttta cttcaagagc tgagggcaaa gcagcctgac 840
ctagctcctg gactgaccac tattggagcc tctcctactc agactgttac tctggtgaca 900
caacctgtgg ttactaagga aactgccatc tccaaactag aaatgccatc ttccttgatg 960
ttggaggtac ctgctctggc agatttcaac cgggcttgga cagaacttac cgactggctt 1020
tctctgcttg atcaagttat aaaatcacag agggtgatgg tgggtgacct tgaggatatc 1080
aacgagatga tcatcaagca gaaggcaaca atgcaggatt tggaacagag gcgtccccag 1140
ttggaagaac tcattaccgc tgcccaaaat ttgaaaaaca agaccagcaa tcaagaggct 1200
agaacaatca ttacggaccg aattgaaaga attcagaatc agtgggatga agtacaagaa 1260
caccttcaga accggaggca acagttgaat gaaatgttaa aggattcaac acaatggctg 1320
gaagctaagg aagaagctga gcaggtctta ggacaggcca gagccaagct tgagtcatgg 1380
aaggagggtc cctatacagt agatgcaatc caaaagaaaa tcacagaaac caagcagttg 1440
gccaaagacc tccgccagtg gcagacaaat gtagatgtgg caaatgactt ggccctgaaa 1500
cttctccggg attattctgc agatgatacc agaaaagtcc acatgataac agagaatatc 1560
aatgcctctt ggagaagcat tcataaaagg gtgagtgagc gagaggctgc tttggaagaa 1620
actcatagat tactgcaaca gttccccctg gacctggaaa agtttcttgc ctggcttaca 1680
gaagctgaaa caactgccaa tgtcctacag gatgctaccc gtaaggaaag gctcctagaa 1740
gactccaagg gagtaaaaga gctgatgaaa caatggcaag acctccaagg tgaaattgaa 1800
gctcacacag atgtttatca caacctggat gaaaacagcc aaaaaatcct gagatccctg 1860
gaaggttccg atgatgcagt cctgttacaa agacgtttgg ataacatgaa cttcaagtgg 1920
agtgaacttc ggaaaaagtc tctcaacatt aggtcccatt tggaagccag ttctgaccag 1980
tggaagcgtc tgcacctttc tctgcaggaa cttctggtgt ggctacagct gaaagatgat 2040
gaattaagcc ggcaggcacc tattggaggc gactttccag cagttcagaa gcagaacgat 2100
gtacataggg ccttcaagag ggaattgaaa actaaagaac ctgtaatcat gagtactctt 2160

gagactgtac gaatatttct gacagagcag cctttggaag gactagagaa actctaccag 2220

gagcccagag agctgcctcc tgaggagaga gcccagaatg tcactcggct tctacgaaag 2280

caggctgagg aggtcaatac tgagtgggaa aaattgaacc tgcactccgc tgactggcag 2340

agaaaaatag atgagaccct tgaaagactc cgggaacttc aagaggccac ggatgagctg 2400

gacctcaagc tgcgccaagc tgaggtgatc aagggatcct ggcagcccgt gggcgatctc 2460

ctcattgact ctctccaaga tcacctcgag aaagtcaagg cacttcgagg agaaattgcg 2520

cctctgaaag agaacgtgag ccacgtcaat gaccttgctc gccagcttac cactttgggc 2580

attcagctct caccgtataa cctcagcact ctggaagacc tgaacaccag atggaagctt 2640

ctgcaggtgg ccgtcgagga ccgagtcagg cagctgcatg aagcccacag ggactttggt 2700

ccagcatctc agcactttct ttccacgtct gtccagggtc cctgggagag agccatctcg 2760

ccaaacaaag tgccctacta tatcaaccac gagactcaaa caacttgctg ggaccatccc 2820

aaaatgacag agctctacca gtctttagct gacctgaata atgtcagatt ctcagcttat 2880

aggactgcca tgaaactccg aagactgcag aaggcccttt gcttggatct cttgagcctg 2940

tcagctgcat gtgatgcctt ggaccagcac aacctcaagc aaaatgacca gcccatggat 3000

atcctgcaga ttattaattg tttgaccact atttatgacc gcctggagca agagcacaac 3060

aatttggtca acgtccctct ctgcgtggat atgtgtctga actggctgct gaatgtttat 3120

gatacgggac gaacagggag gatccgtgtc ctgtctttta aaactggcat catttccctg 3180

tgtaaagcac atttggaaga caagtacaga taccttttca agcaagtggc aagttcaaca 3240

ggattttgtg accagcgcag gctgggcctc cttctgcatg attctatcca aattccaaga 3300

cagttgggtg aagttgcatc ctttgggggc agtaacattg agccaagtgt ccggagctgc 3360

ttccaatttg ctaataataa gccagagatc gaagcggccc tcttcctaga ctggatgaga 3420

ctggaacccc agtccatggt gtggctgccc gtcctgcaca gagtggctgc tgcagaaact 3480

gccaagcatc aggccaaatg taacatctgc aaagagtgtc caatcattgg attcaggtac 3540

aggagtctaa agcactttaa ttatgacatc tgccaaagct gctttttttc tggtcgagtt 3600

gcaaaaggcc ataaaatgca ctatcccatg gtggaatatt gcactccgac tacatcagga 3660

gaagatgttc gagactttgc caaggtacta aaaaacaaat ttcgaaccaa aaggtatttt 3720

gcgaagcatc cccgaatggg ctacctgcca gtgcagactg tcttagaggg ggacaacatg 3780

gaaactcccg ttactctgat caacttctgg ccagtagatt ctgcgcctgc ctcgtcccct 3840

cagctttcac acgatgatac tcattcacgc attgaacatt atgctagcag gctagcagaa 3900

atggaaaaca gcaatggatc ttatctaaat gatagcatct ctcctaatga gagcatagat 3960

gatgaacatt tgttaatcca gcattactgc caaagtttga accaggactc ccccctgagc 4020

cagcctcgta gtcctgccca gatcttgatt tccttagaga gtgaggaaag aggggagcta 4080

gagagaatcc tagcagatct tgaggaagaa aacaggaatc tgcaagcaga atatgaccgt 4140

ctaaagcagc agcacgaaca taaaggcctg tccccactgc cgtcccctcc tgaaatgatg 4200

cccacctctc cccagagtcc ccgggatgct gagctcattg ctgaggccaa gctactgcgt 4260

caacacaaag gccgcctgga agccaggatg caaatcctgg aagaccacaa taaacagctg 4320

gagtcacagt tacacaggct aaggcagctg ctggagcaac cccaggcaga ggccaaagtg 4380

aatggcacaa cggtgtcctc tccttctacc tctctacaga ggtccgacag cagtcagcct 4440

atgctgctcc gagtggttgg cagtcaaact tcggactcca tgggtgagga agatcttctc 4500

agtcctcccc aggacacaag cacagggtta gaggaggtga tggagcaact caacaactcc 4560

ttccctagtt caagaggaag aaatacccct ggaaagccaa tgagagagga cacaatgtag 4620

<210> 12
<211> 3393
<212> DNA
<213> artificial

<220>
<223> Complete sequence for exons 45-52 knock-in target matrix with 1
      kb arms

<400> 12
gggcccacat agttggcacc ggcgaaccat ttttttaaaa aaaatggaac tccaattgta 60

catttattag gctccttaaa gtgccccact actcactgat gttatgttca ttgtctgttt 120

ggtctctctt ttctctgtaa tttgttttat ataatctcta ttgtcaaatt gactaatctt 180

tttcaaagtc taatctatgg ctaatcccat gtagtatata tttttaacat cagacatttt 240

catctcttag aagtaaaagt tgggtctttt tatttcttcc atgtgtctac tcaacatgtt 300

cagtctttac tttcttgact atatggaata cagatataat aactgttaga atattcttct 360

ctactaattt tatcatctgt gtctattctg ggttaattta aattgattta tttttctcct 420

cattaagtgt gttgtttaac tgcttctttg gatgactggt aatttttgac tatatgccag 480

acattgtgaa ttttaactta gcgcatgctt gatacttcaa ataaattcaa atatattgaa 540

ataaatattc tcaaccctcg ttctggaaca cagttaattc acttggaaac aatttgatct 600

tttgagaatc ttccttttat gctttgttat gaccagaaca gtgtaagttt agggctactt 660

tttccccact actgaggcaa aacccttctg agtactctct ctgatgtcct gtgaatgata 720

aaatttttca ctggggctcg tgggaacagg tggtattact agccacgtgt gagctctggt 780

gattgtttcc tttaattctt ttgtgaagtt ctttccttag ctttgagtgg ttttcttgca 840

tacatgaact gatcaagact cagatgaaga ataaaataaa gctttctaca aatctccaaa 900

atttcctctg tgtatatatc acctctctgg tattttgccc tgtgatcact agtcagcctt 960

gggctgctga aactctcagc ttcatctttt aacagaagcc tcctggcaag gatcactgtc 1020

cttcggcgcg cccttgtcga gacagagaag actcttgcgt ttctgatagg cacctattgg 1080

tcttactgac atccactttg cctttctctc cacaggaact gcaggatggc attgggcagc 1140

ggcaaactgt tgtcagaaca ttgaatgcaa ctggggaaga aataattcag caatcctcaa 1200
aaacagatgc cagtattcta caggaaaaat tgggaagcct gaatctgcgg tggcaggagg 1260
tctgcaaaca gctgtcagac agaaaaaaga ggctagaaga acaaaagaat atcttgtcag 1320
aatttcaaag agatttaaat gaatttgttt tatggttgga ggaagcagat aacattgcta 1380
gtatcccact tgaacctgga aaagagcagc aactaaaaga aaagcttgag caagtcaagt 1440
tactggtgga agagttgccc ctgcgccagg gaattctcaa acaattaaat gaaactggag 1500
gacccgtgct tgtaagtgct cccataagcc cagaagagca agataaactt gaaaataagc 1560
tcaagcagac aaatctccag tggataaagg tttccagagc tttacctgag aaacaaggag 1620
aaattgaagc tcaaataaaa gaccttgggc agcttgaaaa aaagcttgaa gaccttgaag 1680
agcagttaaa tcatctgctg ctgtggttat ctcctattag gaatcagttg gaaatttata 1740
accaaccaaa ccaagaagga ccatttgacg ttaaggaaac tgaaatagca gttcaagcta 1800
aacaaccgga tgtggaagag attttgtcta aagggcagca tttgtacaag gaaaaaccag 1860
ccactcagcc agtgaagagg aagttagaag atctgagctc tgagtggaag gcggtaaacc 1920
gtttacttca agagctgagg gcaaagcagc ctgacctagc tcctggactg accactattg 1980
gagcctctcc tactcagact gttactctgg tgacacaacc tgtggttact aaggaaactg 2040
ccatctccaa actagaaatg ccatcttcct tgatgttgga ggtacctgct ctggcagatt 2100
tcaaccgggc ttggacagaa cttaccgact ggctttctct gcttgatcaa gttataaaat 2160
cacagagggt gatggtgggt gaccttgagg atatcaacga gatgatcatc aagcagaagg 2220
caacaatgca ggatttggaa cagaggcgtc cccagttgga agaactcatt accgctgccc 2280
aaaatttgaa aaacaagacc agcaatcaag aggctagaac aatcattacg gaccgaagta 2340
agtatcaagg ttacaagaca ggtttaagga gaccaataga aactgggctc tttctttcca 2400
tgggttggcc tacgcgtaag tgtttcaggt gtttcaggca ggagattaag ttcagcctcc 2460
tttactccaa cttgaaaaca agtccaaaac aaactatttt gatgtaattt gatcttttaa 2520
tacattaaca ttacacaatt ttgtgaatat atcataattt aaaattttca gagaatgtct 2580
aatggtcctc atttcttgac agtgtggttt agttgaaact gatgaacatt ttatcaaaac 2640
ttttcccctc aattggatac tttttttttt ttgagatgga attttgcttt tgtcacccag 2700
gctggagtgg catgatctca gctcactgca acctctgcct ccaggcttca agcaattctc 2760
ctgccttagc ctcccgagta gctgggatta caggtgccca cccccacacc tggctaattt 2820
ttgtattttt agtagagacg agatttcacc atgttggtca ggctggtcta gatctccgac 2880
ctcaggtggt ctgcctgtct cagcctccca aagtgctggg attgcagacg tgagccacca 2940
tgcctggcca actggataat ttttaaaaga ccattttatt tagtctattt tttctcaatc 3000
tatagatgag ataagaaaaa tcattctaga tgtccaagga aaaattcttt cagaaaagag 3060

ctgtgaatga tatcacaaac cccccaaaca gttaaggtat ttctttcctg gttattttat 3120

gtccaaaatc atgcatatga acatgtgcac acacatgagc gtgcacacac acatgaatac 3180

atatacacgc acataatgta ccttaggtta tctttccatt ctgagtaatt atcgtaaaat 3240

gggtaaaatc aaccccgtaa gataccttca tcgataaggc aaatcaaagc tttggtaatt 3300

tctgctatct tggcctttgt tgattgacta ataatgaata agagaatgag tttcaatatt 3360

tactatgaag gccggccaac ggatacaacc ggt 3393

<210> 13
<211> 4441
<212> DNA
<213> artificial

<220>
<223> Complete sequence for exons 45-52 knock-in target matrix with 1.5
      kb arms

<400> 13
gggcccacat agttggcacc ggcgtgcttt aatatagaag atacatttta aaaatagcac 60

tgaagtgaat tttgggctaa tgtagcataa tggggtttct gcctgagagg cagaaacata 120

ttagagttat ataaaatgtt ttggggtaga tatagaaacc acttgccatt ttcaatgata 180

tccaacccaa ggtagttata tatttcaatt tatattttat tatcaaatta gtacttattg 240

tgaaaaaaat caagtaacat agaaatttgt aaaagtacct ccattctact ctttggagga 300

tagttgttca gtatgaattt tgctacatat ttcaggctgg gtttcttgga aagccattgt 360

aaaatggaga tttgtatgta gaaggttaac tagggagtac ttttacgatg aagcaatttg 420

ttttgatgta acttggtgta gttttcttca tgtttcttgt tcttgaagtc agttaagctc 480

ttgaatctgt gcatttaaca tttcatcaaa tttagaaacc tttcaaccat ttttttaaaa 540

aaaatggaac tccaattgta catttattag gctccttaaa gtgccccact actcactgat 600

gttatgttca ttgtctgttt ggtctctctt ttctctgtaa tttgttttat ataatctcta 660

ttgtcaaatt gactaatctt tttcaaagtc taatctatgg ctaatcccat gtagtatata 720

tttttaacat cagacatttt catctcttag aagtaaaagt tgggtctttt tatttcttcc 780

atgtgtctac tcaacatgtt cagtctttac tttcttgact atatggaata cagatataat 840

aactgttaga atattcttct ctactaattt tatcatctgt gtctattctg ggttaattta 900

aattgattta tttttctcct cattaagtgt gttgtttaac tgcttctttg gatgactggt 960

aatttttgac tatatgccag acattgtgaa ttttaactta gcgcatgctt gatacttcaa 1020

ataaattcaa atatattgaa ataaatattc tcaaccctcg ttctggaaca cagttaattc 1080

acttggaaac aatttgatct tttgagaatc ttccttttat gctttgttat gaccagaaca 1140

gtgtaagttt agggctactt tttccccact actgaggcaa aacccttctg agtactctct 1200

ctgatgtcct gtgaatgata aaatttttca ctggggctcg tgggaacagg tggtattact 1260

agccacgtgt gagctctggt gattgtttcc tttaattctt ttgtgaagtt ctttccttag 1320

ctttgagtgg ttttcttgca tacatgaact gatcaagact cagatgaaga ataaaataaa 1380

gctttctaca aatctccaaa atttcctctg tgtatatatc acctctctgg tattttgccc 1440

tgtgatcact agtcagcctt gggctgctga aactctcagc ttcatctttt aacagaagcc 1500

tcctggcaag gatcactgtc cttcggcgcg cccttgtcga gacagagaag actcttgcgt 1560

ttctgatagg cacctattgg tcttactgac atccactttg cctttctctc cacaggaact 1620

gcaggatggc attgggcagc ggcaaactgt tgtcagaaca ttgaatgcaa ctggggaaga 1680

aataattcag caatcctcaa aaacagatgc cagtattcta caggaaaaat tgggaagcct 1740

gaatctgcgg tggcaggagg tctgcaaaca gctgtcagac agaaaaaaga ggctagaaga 1800

acaaaagaat atcttgtcag aatttcaaag agatttaaat gaatttgttt tatggttgga 1860

ggaagcagat aacattgcta gtatcccact tgaacctgga aaagagcagc aactaaaaga 1920

aaagcttgag caagtcaagt tactggtgga agagttgccc ctgcgccagg gaattctcaa 1980

acaattaaat gaaactggag gacccgtgct tgtaagtgct cccataagcc cagaagagca 2040

agataaactt gaaaataagc tcaagcagac aaatctccag tggataaagg tttccagagc 2100

tttacctgag aaacaaggag aaattgaagc tcaaataaaa gaccttgggc agcttgaaaa 2160

aaagcttgaa gaccttgaag agcagttaaa tcatctgctg ctgtggttat ctcctattag 2220

gaatcagttg gaaatttata accaaccaaa ccaagaagga ccatttgacg ttaaggaaac 2280

tgaaatagca gttcaagcta aacaaccgga tgtggaagag attttgtcta aagggcagca 2340

tttgtacaag gaaaaaccag ccactcagcc agtgaagagg aagttagaag atctgagctc 2400

tgagtggaag gcggtaaacc gtttacttca agagctgagg gcaaagcagc ctgacctagc 2460

tcctggactg accactattg gagcctctcc tactcagact gttactctgg tgacacaacc 2520

tgtggttact aaggaaactg ccatctccaa actagaaatg ccatcttcct tgatgttgga 2580

ggtacctgct ctggcagatt tcaaccgggc ttggacagaa cttaccgact ggctttctct 2640

gcttgatcaa gttataaaat cacagagggt gatggtgggt gaccttgagg atatcaacga 2700

gatgatcatc aagcagaagg caacaatgca ggatttggaa cagaggcgtc cccagttgga 2760

agaactcatt accgctgccc aaaatttgaa aaacaagacc agcaatcaag aggctagaac 2820

aatcattacg gaccgaagta agtatcaagg ttacaagaca ggtttaagga gaccaataga 2880

aactgggctc tttctttcca tgggttggcc tacgcgtaag tgtttcaggt gtttcaggca 2940

ggagattaag ttcagcctcc tttactccaa cttgaaaaca agtccaaaac aaactatttt 3000

gatgtaattt gatcttttaa tacattaaca ttacacaatt ttgtgaatat atcataattt 3060

aaaattttca gagaatgtct aatggtcctc atttcttgac agtgtggttt agttgaaact 3120

gatgaacatt ttatcaaaac ttttcccctc aattggatac tttttttttt ttgagatgga 3180

attttgcttt tgtcacccag gctggagtgg catgatctca gctcactgca acctctgcct 3240

ccaggcttca agcaattctc ctgccttagc ctcccgagta gctgggatta caggtgccca 3300

cccccacacc tggctaattt ttgtattttt agtagagacg agatttcacc atgttggtca 3360

ggctggtcta gatctccgac ctcaggtggt ctgcctgtct cagcctccca aagtgctggg 3420

attgcagacg tgagccacca tgcctggcca actggataat tttaaaaaga ccattttatt 3480

tagtctattt tttctcaatc tatagatgag ataagaaaaa tcattctaga tgtccaagga 3540

aaaattcttt cagaaaagag ctgtgaatga tatcacaaac cccccaaaca gttaaggtat 3600

ttctttcctg gttattttat gtccaaaatc atgcatatga acatgtgcac acacatgagc 3660

gtgcacacac acatgaatac atatacacgc acataatgta ccttaggtta tctttccatt 3720

ctgagtaatt atcgtaaaat gggtaaaatc aaccccgtaa gataccttca tcgataaggc 3780

aaatcaaagc tttggtaatt tctgctatct tggcctttgt tgattgacta ataatgaata 3840

agagaatgag tttcaatatt tactatgaaa ttattttaga agacaggatg tagacagtgg 3900

ctgttagcag gcaattgttt ggcatgagcc agtaatggtt actgtgaaaa aaatcaacca 3960

agcagcccat atattaaaca aacacacgca gaagcacgtt ggagtctgaa gcctcatatg 4020

tacaattttc agtaaagaaa taacttttag atatgaaata aacaaataga tatatgttgt 4080

aaacttgtcc ctatgtattt tgatcaaatt gcatcatatt tttttcactt taaagaagag 4140

aatttagtgc tttaactgag acttagtgtt atcattcaaa atatactgac tgccaatagc 4200

agcagaaaga taatctggtt ccatgcaact ctattttttt tcctctgtcg caagtaaaag 4260

acaaaattaa gtacatgaat tagtgctttt tgaagatatt ccagagcaat ataccatgcc 4320

actatggaga acctctctaa aaatatccca tttttttacc tgagaaaaat attgatcatg 4380

ttatatgcca ctcaaattgg tttattaaat tcgttgaggc cggccaacgg atacaaccgg 4440

t 4441

<210> 14
<211> 20
<212> DNA
<213> artificial

<220>
<223> DMD31 for3 primer

<400> 14
agagggaagc atcgtaacag 20

<210> 15
<211> 22
<212> DNA
<213> artificial

<220>
<223> KI h45 rev primer

<400> 15

cttttagttg ctgctctttt cc                                                22

<210>  16
<211>  21
<212>  DNA
<213>  artificial

<220>
<223>  DMD31 for4 primer

<400>  16
tggtgtcctt tgaatatgca g                                                 21

<210>  17
<211>  21
<212>  DNA
<213>  artificial

<220>
<223>  h51f primer

<400>  17
ttaccgactg gctttctctg c                                                 21

<210>  18
<211>  21
<212>  DNA
<213>  artificial

<220>
<223>  DMD31-Rev12 primer

<400>  18
caattcctaa tccaagagcc c                                                 21

<210>  19
<211>  19
<212>  DNA
<213>  artificial

<220>
<223>  DMD31-Rev11 primer

<400>  19
gagcctcatg tggattgtc                                                    19

<210>  20
<211>  20
<212>  DNA
<213>  artificial

<220>
<223>  h43f primer

<400>  20
acaacaaagc tcaggtcgga                                                   20

<210>  21
<211>  22

<212>  DNA
<213>  artificial

<220>
<223>  h48r primer

<400>  21
ctgctcttga aggtcttcaa gc                                                22


<210>  22
<211>  22
<212>  DNA
<213>  artificial

<220>
<223>  h44f primer

<400>  22
cagtggctaa cagaagctga ac                                                22


<210>  23
<211>  21
<212>  DNA
<213>  artificial

<220>
<223>  h46r primer

<400>  23
ccaggttcaa gtgggatact a                                                 21


<210>  24
<211>  20
<212>  DNA
<213>  artificial

<220>
<223>  h50f primer

<400>  24
aagcagcctg acctagctcc                                                   20


<210>  25
<211>  22
<212>  DNA
<213>  artificial

<220>
<223>  h55r primer

<400>  25
gttgtttcag cttctgtaag cc                                                22


<210>  26
<211>  20
<212>  DNA
<213>  artificial

<220>
<223>  h54r primer

<400> 26
gtctgccact ggcggaggtc 20

## Description

The present invention relates to a gene therapy method for the treatment of Duchenne muscular dystrophy, or DMD.

### BACKGROUND OF THE INVENTION

Duchenne muscular dystrophy (DMD), affecting 1 in 3500 male births is caused by nonsense or frameshift mutations in the gene encoding dystrophin, resulting in the absence of dystrophin protein (Muntoni et al., 2003, Lancet Neurol 2: 731-40). Dystrophin is required for the assembly of the dystrophin-glycoprotein complex, and provides a mechanically strong link between the cytoskeleton and the extracellular matrix (Gumerson et al., 2011, J Biomed Biotechnol 2011: 210797). Dystrophin-deficient DMD muscle is therefore mechanically destabilized and this is the primary cause of the myofibre necrosis and muscle wasting seen in this lethal disease (Matsumura et al., 1993, Neuromuscul Disord 3: 533-5). There is currently no gene repair therapy available for DMD; conventional therapies are limited to supportive care which partially alleviates signs and symptoms, but does not directly target the disease mechanism, nor reverse the phenotype. There are currently several gene therapy strategies being developed for DMD including in vivo gene therapy with adeno-associated virus (AAV) vectors, cell transplantation therapy, pharmacologic rescue of DMD nonsense mutations and exon skipping strategies to repair the DMD gene reading frame. All of these strategies have problems to overcome, including targeting different muscle groups, optimization of delivery, long-term expression of the transgene, and potential immune response.

Gene targeting is a powerful tool for creating genetic modifications as a gene repair strategy for a variety of genetic diseases (Carroll et al., 2011, Genetics 188: 773-82; Jensen et al., 2011, J Biomed Sci 18:10; Martin et al., 2010, Clin Cancer Res 16: 5107-13). Several means are known for achieving genome surgery. For example, one can use endonucleases such as zinc finger nucleases (ZFNs), meganucleases (MNs), and transcription activator-like effector nucleases (TALENs) to cut at specific site in the DNA. The resulting double strand break (DSB) can be repaired in two major ways; by homologous recombination (HR) or non-homologous end joining (NHEJ) (Longhese et al., 2010, EMBO J 29: 2864-74). HR requires the presence of an identical or nearly identical sequence to be used as a template for repair of the break (Jensen et al., 2011, J Biol Chem 18: 10-14). For example, MNs can be used to stimulate homologous combination (HR) up to 10000-fold in cultured cells (Rouet et al., 1994, Mol Cell Biol 14: 8096-8106; Choulika et al., 1995, Mol Cell Biol 15: 1968-1973) in comparison to homologous recombination at a non-cleaved site. MNs have been used to induce HR in a variety of cell types and organisms (Paques et al., 2007, Curr Gene Ther 7: 49-66) including mammalian cells, mice, plants, Drosophila, E. coli and trypanosome Boothroyd et al., 2009, Nature 459: 278-281). Efficient gene targeting of the human XPC gene with MNs (Arnoud et al., 2007, J Mol Biol 371: 49-65), and ZFN and HR-mediated gene repair of the severe combined immune deficiency (SCID) mutation on the IL2Ry gene (Umov et al., 2005, Nature 435: 646-651; Lombardo et al., 2007, Nature Biotech 25: 1298-1306), and the homology-directed targeted gene correction of the haemophilia mutation on the F9 gene to restore haemostasis (Li et al., 2011, Nature 475: 217-221), have all been recently demonstrated. Of course, as mentioned above, besides MNs other endonucleases may be used to achieve efficient gene targeting.

In the context of DMD, genome surgery has been limited to a proof-of-principle study in which an engineered MN restored the normal reading frame of dog micro-dystrophin sequences carrying a frameshift mutation through NHEJ repair. The MN induced micro-deletion or micro-insertion (INDELs) in the micro-dystrophin so that dystrophin expression was restored, both in myoblasts in vitro and in muscle fibres in vivo (Chapdelaine et al., 2010, Gene Ther 17: 846-858). This study reports the use of an endonuclease to restore the reading frame of a gene by NHEJ, rather than genome correction via HR.

The present inventors demonstrate for the first time targeted cDNA knock-in and DMD gene repair in vitro in DMD patient cells through HR repair of an endonuclease-induced DSB by a targeting repair matrix. The work presented here demonstrates that endonuclease-enhanced genome correction therapy for DMD is possible.

### SUMMARY OF THE INVENTION

The invention relates to the treatment of Duchenne muscular dystrophy (DMD) by dystrophin gene repair through homologous recombination.

More specifically, the invention relates to a nucleic acid construct comprising a first and second portions which are homologous to regions 5' and 3' of a dystrophin gene sequence of interest, further comprising a third portion positioned between the first and second portion, said third portion comprising a dystrophin correcting cDNA flanked either side by splice donor and acceptor sites or other appropriate regulatory sequences (e.g. translational terminator and polyadenylation sites). This nucleic acid is used as a repair matrix for effecting permanent genome correction at the dystrophin gene upon cutting of the genomic target site, for example by the engineered nuclease. The construct may be introduced as a DNA fragment, into a plasmid or in the genome of a viral vector. Said nucleic acid construct, plasmid or viral vector is used in a method for repairing the dystrophin gene in the genome of a target cell. Specifically, an endonuclease is used to induce a double-strand break (DSB) at a locus of interest in the dystrophin gene, which is repaired through homologous recombination at the site of DSB thanks to the nucleic acid construct of the invention, thereby resulting in the knock-in of missing exonic cDNA into the genome of a target cell.

More specifically, we herein disclose a nucleic acid construct for the repair of the dystrophin gene in cells having a deletion of exons 45 to 52 of said gene.

These and other embodiments, features and advantages of the disclosure will become apparent from the detailed description and the appended claims set forth herein below.

### LEGEND OF THE FIGURES

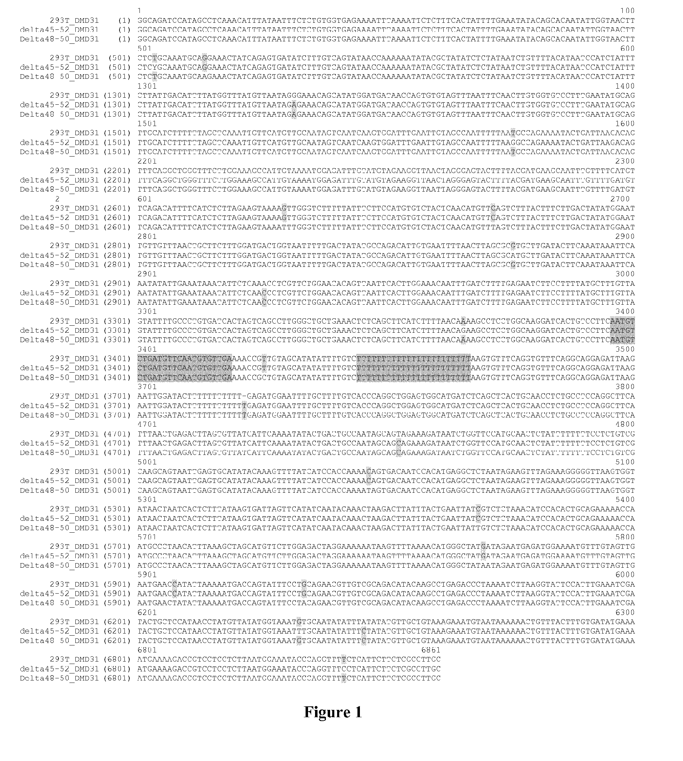

Figure 1: Intron 44 sequence around MN DMD31 target site +/- ∼3kb on 293T, del48-50 and del45-52 genomic DNA
   Residues showing polymorphism are highlighted, and the MN-DMD31 target site is highlighted gray (positions 3396 - 3419). It should be noted that although a number of extra primers were specifically designed and used for sequencing, the number of residues in the poly(T) sequence (highlighted in dark gray) just downstream from the target site could not be resolved. The arm of homology was therefore designed to be downstream of this poly(T) sequence.

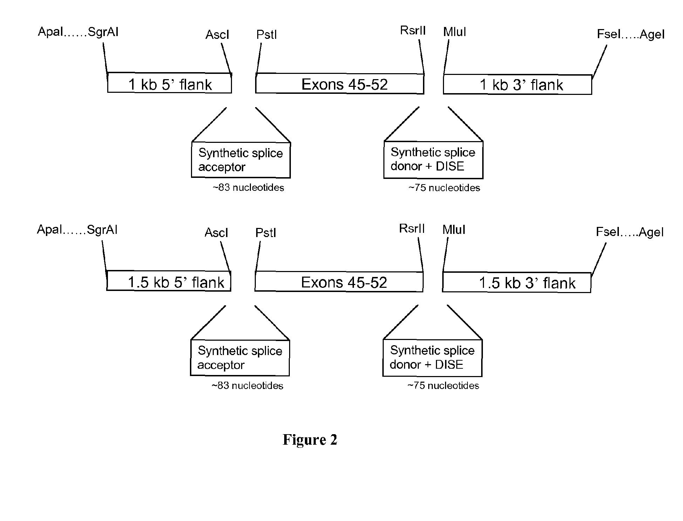

Figure 2: Schematic RE map repair matrices S1 (a) and S2 (b) for targeting exon 45-52 deleted genotypes
   The exon 45-52 cDNA block with flanking artificial splice sites (synthetic branch point, polypyrimidine tract and splice acceptor site, synthetic splice donor and intronic splicing enhancer sequence from rat FGFR2 gene (DISE element)) and arms of homology have been incorporated into repair matrices as shown.
Figure 3: Homologous recombination in LV-endonuclease (MN) + IDLV-matrix (S2) transduced cells at genomic DNA level.
Figure 4: Restoration of full-length dystrophin mRNA in human del45-52 DMD cells by knock-in of exons 45 to 52 by homologous recombination of the targeting matrix (S2) upon endonuclease (MN) cutting.
Figure 5: Confirmation that knocked-in exons are correctly spliced to neighbouring endogenous exons in RNA.
Figure 6: diagram of a basic matrix building block showing the different elements of the construct and restriction sites.
Figure 7a: diagram illustrating the replacement of the synthetic acceptor present in the matrix of figure 6 by an endogenous (or other) splice acceptor.
Figure 7b: diagram illustrating the replacement of the synthetic splice donor present in the matrix of figure 7a by an endogenous (or other) splice donor.
Figure 7c: diagram illustrating the modification of the length of the arms of homology.
Figure 7d: diagram illustrating the replacement of the cDNA block with a block of exons 45 to 79 and 1 kb arms of homology.
Figure 7e: diagram illustrating the change of the length of the arms of homology to 3 kb within the matrix of figure 7d comprising a cDNA block encoding exons 45 to 79.

### DETAILED DESCRIPTION OF THE INVENTION

The present invention provides a nucleic acid construct comprising a first and second portions which are homologous to regions 5' and 3' of a dystrophin gene sequence of interest, further comprising a third portion positioned between the first and second portion, said third portion comprising a dystrophin correcting cDNA flanked either side by splice donor and acceptor sites or other appropriate elements (e.g. translational terminator and polyadenylation sites).

The practice of the present invention will employ, unless otherwise indicated, conventional methods of virology, microbiology, molecular biology and recombinant DNA techniques within the knowledge of those skilled in the art. Such techniques are explained fully in the literature. See, e.g., Sambrook, et al. Molecular Cloning: A Laboratory Manual (Current Edition); DNA Cloning: A Practical Approach, vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijssen, ed.); Fundamental Virology, 2nd Edition, vol. I & II (B. N. Fields and D. M. Knipe, eds.)
The various compositions and methods of the invention are described in detail below. Although particular compositions and methods are exemplified herein, it is understood that any of a number of alternative compositions and methods are applicable and suitable for use in practicing the invention.

### Definitions

Unless otherwise indicated, all terms used herein have the same meaning as they would to one skilled in the art, and the practice of the present invention will employ conventional techniques of microbiology and recombinant DNA technology, which are within the knowledge of those of skill in the art.

The term "nucleic acid construct" refers generally to a nucleic acid which is comprised of segments joined together using recombinant DNA technology.

The term "recombinant" as used herein refers to nucleic acids, vectors, polypeptides, or proteins that have been generated using DNA recombination (cloning) methods and are distinguishable from native or wild-type nucleic acids, vectors, polypeptides, or proteins.

The term "subject" as used herein includes, but is not limited to, humans, nonhuman primates such as chimpanzees and other apes and monkey species; farm animals such as cattle, sheep, pigs, goats and horses; domestic mammals such as dogs and cats; laboratory animals including rodents such as mice, rats and guinea pigs, and the like. The term does not denote a particular age or sex. Thus, adult and newborn subjects, as well as fetuses, whether male or female, are intended to be covered.

The term "therapeutically effective amount" refers to an amount effective, at dosages and for periods of time necessary, to achieve the desired therapeutic result. A therapeutically effective amount of the recombinant nucleic acid of viral vector may vary according to factors such as the disease state, age, sex, and weight of the individual and the ability of the vector to elicit a desired response in the individual. A therapeutically effective amount is also one in which any toxic or detrimental effect is outweighed by the therapeutically beneficial effects.

As used herein, "ex vivo administration" refers to a process where primary cells or an entire organ are harvested from a subject, a nucleic acid or viral vector is delivered into the cells, and the cells are readministered to the same or a different subject.

In the present description, any concentration range, percentage range, ratio range, or integer range is to be understood to include the value of any integer within the recited range and, when appropriate, fractions thereof (such as one tenth and one hundredth of an integer), unless otherwise indicated. Also, any number range recited herein relating to any physical feature, such as polymer subunits, size or thickness, are to be understood to include any integer within the recited range, unless otherwise indicated.

The term "about" or "approximately" means within a statistically meaningful range of a value. Such a range can be within an order of magnitude, preferably within 50%, more preferably within 20%, more preferably still within 10%, and even more preferably within 5% of a given value or range. The allowable variation encompassed by the term "about" or "approximately" depends on the particular system under study, and can be readily appreciated by one of ordinary skill in the art.

### Repair matrix

The nucleic acid construct of the invention is provided as a repair matrix for the correction of the dystrophin gene in the genome of a target cell of a subject in need thereof.

This nucleic acid construct comprises three portions. The first and second portions, which are also collectively referred to as "arms of homology" are homologous to regions 5' and 3' of a dystrophin gene sequence of interest and a third portion flanked either side by splice donor and acceptor sites or other appropriate elements (e.g. translational terminator and polyadenylation sites), positioned between the first and second portions, comprising a dystrophin correcting cDNA.

The first and second portions are thus homologous to a region of interest in the dystrophin gene. These regions of interest are selected among known mutation hotspots (Tuffery-Giraud et al., 2009, Hum Mutat 30: 934-945). More specifically, one may cite mutational hotspots including and spanning intron 38 to 52 and intron 2-9 of the dystrophin gene. In a particular embodiment, the nucleic acid construct of the present invention comprises nucleic acid sequences homologous to part of intron 44. More than 25 % dystrophin mutations arise from this specific intron.

The first and second portions are more particularly homologous to sequences found either side of a double-strand break-inducing endonuclease target site in the selected dystrophin gene sequence of interest. By "homologous" is intended a sequence with enough identity to another one to lead to a homologous recombination between sequences, more particularly having at least 95 % identity, preferably 97 % identity and more preferably 99 %. "Identity" refers to sequence identity between two nucleic acid molecules. Identity can be determined by comparing a position in each sequence which may be aligned for purposes of comparison. When a position in the compared sequences is occupied by the same base, then the molecules are identical at that position. A degree of similarity or identity between nucleic acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.

In a particular embodiment, the sequence of interest is in intron 44, and the target site is located within intron 44, 2.233 kb downstream from exon 44, at position *>chromosome:GRCh37:X:32364354:32364377,* and has the sequence AATGTCTGATGTTCAATGTGTTGA (SEQ ID NO:1). In a specific variant, the first and second portions are identical to the sequences present either side (e.g. within 100bp) of the endonuclease target site in the genomic sequence of interest.

The first and second portions are independently from each other 0.5 kb to 8 kb in length, more particularly 1 kb to 3 kb in length. For example, the arms of homology may each be 1 kb, 1.5 kb or 3 kb in length. Illustrative left arms of homology are shown in SEQ ID NOs: 2 and 3, and illustrative right arms of homology are shown in SEQ ID NOs: 4 and 5.

The third portion is a cDNA block, including dystrophin exons to be introduced in the genome of the target cells. Representative third portions include portions comprising exons 45-52 of the dystrophin gene (see for example the sequence shown in SEQ ID NO:10), or even the full 45-79 exons (see for example the sequence shown in SEQ ID NO:11), or any therapeutically effective intermediate selection of exons.

In addition to the first, second and third portions, the nucleic acid construct of the present invention comprises regulatory sequences flanking said third portion. In a particular embodiment, the regulatory sequences are a splice acceptor (e.g. the synthetic splice acceptor of SEQ ID NO:6) and a splice donor sequences located respectively 5' and 3' of the third portion. As a result, the construct of the invention may comprise, in the 5' to 3' direction, the first portion, a splice acceptor site, the third portion, a splice donor site and the second portion. The splice sites may either be endogenous (i.e. naturally-occurring in the wild-type dystrophin gene) or synthetic (i.e. non-natural sequences designed in silico; see for example Shapiro et al., Nucl Acids Res, 15: 7155-7174). In a particular embodiment, a synthetic splice donor site is provided together with an intronic splicing enhancer sequence, for example the intronic splicing enhancer sequence from rat FGFR2 gene (also herein referred to as DISE element). Sequence of such splice donor site with a DISE element is shown in SEQ ID NO:7. In an alternative variant, the third portion of the nucleic acid construct is flanked by other regulatory sequences. For example, the third portion may be flanked in 5' by a splice acceptor site and in 3' by a WPRE sequence (Woodchuck Post-transcriptional Regulatory Element; included to enhance expression of the cDNA block - see for example the sequence shown in SEQ ID NO:9) fused to a poly(A) sequence when said third portion is the full 45-79 exons, for example the bovine growth hormone poly(A) sequence (see for example SEQ ID NO:8).

According to a specific embodiment, the nucleic acid construct of the invention comprises a cDNA block comprising exons 45 to 52 of the dystrophin gene (third portion), flanked arms of homology to intron 44 (first and second portions), synthetic splice donor and acceptor and an intronic splicing enhancer sequence (e.g. from rat FGFR2 gene). Such nucleic acid constructs are shown in SEQ ID NO:12 and 13.

The nucleic acid construct of the invention may further comprise various unique restriction enzyme (RE) sites incorporated between each element of said construct to allow its modification in a stepwise manner. As an illustrative, non-limiting example, when the third portion contains exons 45-52 of the dystrophin gene, a PstI restriction site may be silently introduced at the start of exon 45 by a single point mutation of the second codon of said exon (CTC replaced by a CTG thereby providing a CTGCAG PstI restriction site) and a RsrII restriction site may be introduced at the end of exon 52 by a single point mutation of the penultimate codon of said exon (GAT replaced by a GAC thereby providing a CGACCG RsrII restriction site). Other unique restriction sites may be provided on the construct. Figures 2, 6 and 7a-e illustrate a number of possible restriction sites that may be introduced in a nucleic acid construct of the invention. The restrictions sites incorporated into the nucleic acid construct of the invention may allow various modifications such as: (i) introduction and modification of the splice donor and acceptor; (ii) modification of the length of the arms of homology; (iii) replacing the cDNA block of the third portion (for example introducing the full 45-79 exons when the arms of homology are specific of intron 44); (iv) introduction of a transcriptional terminator in 3' of the full 45-79 exons; (v) introduction of positive and/or negative selection markers if required.

The nucleic acid construct of the invention may be introduced in a plasmid vector or a viral vector. Accordingly, the invention also relates to a recombinant plasmid comprising the nucleic acid construct described above, and to a recombinant viral vector comprising in its genome the nucleic acid construct of the invention. Plasmid and viral vector design is known to those skilled in the art. A viral vector of the invention may be any viral vector efficient for transducing a cell involved in a dystrophin-related disease (a target cell). In particular, the target cell may be a muscle cell such as a myoblast, mesoangioblast, pericyte, or CD133+ or CD56+ progenitor cells. Illustrative, non-limiting, viral vectors useful for the invention include adenovirus, adeno-associated virus and lentivirus vectors. For example, as provided in the examples, the nucleic acid construct may be introduced in an integration-deficient lentiviral vector, in particular pseudotyped with the the VSV-G envelope glycoprotein. The construct may in particular be inserted invertedly in a lentiviral backbone such as the pRRLsin.PPT plasmid. Lentiviral vectors are produced according to methods well known in the art. For example, plasmids carrying all necessary elements of a lentivirus are transiently transfected in a mammalian cell (e.g. HEK293T cells), then after a period of 16 h to 72 h, in particular 40 h, the cell medium is collected, centrifuged and lentivirus vectors are purified from the supernatant. The person skilled in the art will refer to the examples provided below, and to his general knowledge in this field as represented in particular by Sakumo et al., 2012 Biochem J 443: 603-618.

### Administration of the repair matrix

The target cells the genome of which is to be corrected by the nucleic acid construct of the invention can be derived from a human, and other mammals such as primates, horse, sheep, goat, pig, dog, rat, and mouse. Examples of target cells to which the construct can be delivered include, but are not limited to, muscle cells, central nervous system cells and peripheral nervous system cells. Examples of tissues to which the construct can be delivered include muscle, heart, eye and brain.

For *in vitro, ex vivo* or *in vivo* delivery to cells, tissues or organs of a subject, the nucleic acid construct, recombinant plasmid or recombinant viral vector of the invention may be incorporated into compositions, in particular pharmaceutical compositions suitable for administration to a subject. Typically, the pharmaceutical composition comprises a pharmaceutically acceptable carrier and a nucleic acid construct, recombinant plasmid or recombinant viral vector of the invention. The term "pharmaceutically acceptable" as used herein refers to molecular entities and compositions that are physiologically tolerable and do not typically produce toxicity or an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. or European Pharmacopeia, or other generally recognized pharmacopeia for use in animals, and more particularly in humans. Also, as used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. Pharmaceutical compositions can be delivered as, for example, ageratum, sprays, oral suspensions, suppositories, eye drops, and injectable suspensions.

The pharmaceutical composition may be suitable for topical, systemic, intra-amniotic, intrathecal, intracranial, intraarterial, intravenous, intralymphatic, intraperitoneal, subcutaneous, tracheal, intra-tissue (e.g., intramuscular, intracardiac, intrahepatic, intrarenal, intracerebral), intrathecal, intravesical, conjunctival (e.g., extra-orbital, intraorbital, retroorbital, intraretinal), and mucosal (e.g., oral, rectal, nasal) administration. Passive tissue transduction via high pressure intravenous or intraarterial infusion, as well as intracellular injection, such as intranuclear microinjection or intracytoplasmic injection, are also contemplated.

Pharmaceutical compositions for therapeutic purposes typically must be sterile and stable under the conditions of manufacture and storage. The composition can be formulated as a solution, microemulsion, dispersion, liposomes, or other ordered structure suitable to high product concentration. Sterile injectable solutions can be prepared by incorporating the construct, recombinant plasmid or recombinant viral vector in the required amount in an appropriate buffer with one or a combination of ingredients enumerated above, as required, followed by filtered sterilization.

For in vivo administration, broad distribution can be achieved in muscle by, for example, intravenous, intra-arterial injection or hydrodynamic locoregional perfusion. Depending on dosage and mode of delivery, 20-95% of muscle fibres in a given target muscle can be transduced. Broad distribution of the nucleic acid construct, plasmid or viral vector of the invention can also be achieved in the CNS or PNS by injection into the cerebrospinal fluid, e.g., by lumbar puncture (e.g., Kapadia et al., 1996). Alternatively, precise delivery of the vector into specific sites of the brain to target a neural cell, can be conducted using stereotactic microinjection techniques (Snyder-Keller et al., 2010). Particularly preferred delivery methods are those that deliver the nucleic acid construct, plasmid or viral vector to specific regions of the brain or muscle that require correction of the dystrophin gene. Direct injection, whether subcutaneous, intracranial, intramuscular, or intradermal, can take place using standard needle and syringe methodologies, or by needle-free technologies.

Further methods for delivery of nucleic acid molecules, are described, for example, in Boado et al., J. Pharm. Sci. 87:1308-1315 (1998); Tyler et al., FEBS Lett. 421:280-284 (1999); Pardridge et al., Proc. Nat'l Acad. Sci. USA 92:5592-5596 (1995); Boado, Adv. Drug Delivery Rev. 15:73-107 (1995); Aldrian-Herrada et al., Nucleic Acids Res. 26:4910-4916 (1998); Tyler et al., Proc. Nat'l Acad. Sci.USA 96:7053-7058 (1999); Akhtar et al., Trends Cell Bio. 2:139 (1992); "Delivery Strategies for Antisense Oligonucleotide Therapeutics," (ed. Akhtar, 1995); Maurer et al., Mol. Membr.Biol. 16:129-140 (1999); Hofland and Huang, Handb. Exp. Pharmacol 137:165-192 (1999); and Lee et al., ACS Symp. Ser. 752:184-192 (2000).

Appropriate doses will depend on the particular cell type, tissue, organ, or subject being treated. When administered to a subject, dose will depend on the particular mammal being treated (e.g., human or nonhuman primate or other mammal), age and general condition of the subject to be treated, the severity of the condition being treated, the particular therapeutic polypeptide, protein, or oligonucleotide in question, its mode of administration, among other factors. An appropriate effective amount can be readily determined by one of skill in the art.

A "therapeutically effective dose" will fall in a relatively broad range that can be determined through clinical trials and will depend on the particular application (neural cells will require very small amounts, while systemic injection would require large amounts).

Treatment may involve administration of a single dose or multiple doses, depending in particular on the severity of the disease.

### Therapeutic methods of the invention

The nucleic acid construct of the invention is particularly useful for restoring dystrophin expression in a target cell. The invention thus relates to a method of treatment of a disease caused by a mutation in the dystrophin gene, comprising administering to a subject in need thereof a nucleic acid construct, recombinant plasmid or recombinant vector of the invention. According to another embodiment, the invention relates to a method for restoring full-length dystrophin expression in a subject in need thereof. The invention also relates to a method for correcting the expression of dystrophin in a subject in need thereof. The invention further relates to a method for altering the genomic DNA sequence of the dystrophin gene in a target cell. Moreover, the invention relates to a method for repairing the dystrophin gene in the genome of a target cell. The invention also relates to a method for inserting a cDNA block of missing exons in the dystrophin gene in the genome of a target cell. Representative diseases treatable with the invention include Becker and Duchenne muscular dystrophies. In a preferred embodiment, the invention relates to a method of treatment of Duchenne muscular dystrophy, comprising administering to a subject in need thereof a nucleic acid construct, recombinant plasmid or recombinant vector of the invention.

In a particular embodiment, the subject presents a deletion of dystrophin exons 45 to 52 in its genome. Indeed, the below examples show correction of deletion of exons 45-52 in patient cells by knock-in of cDNA encoding exons 45-52, following DNA cleavage by an endonuclease. The nucleic acid construct of the invention thus may be used to correct the dystrophin gene in up to a quarter of Duchenne muscular dystrophy patients by cDNA knock-in through homologous recombination repair of endonuclease-driven double strand break (DSB) in intron 44 of the DMD gene.

The methods of the present invention are implemented as follows:
- cleavage of genomic dystrophin gene is induced at a sequence of interest; and
- the nucleic acid construct as defined above is provided as a template for homologous recombination at the cleavage site.

The cleavage in the dystrophin gene may be provided by different means. In a particular embodiment, said cleavage is induced in the sequence of interest by the use of a nickase or a double-strand break-inducing endonuclease. Alternatively, small molecules that damage DNA or create altered structures that may induce homologous recombination and can be targeted to specific sequences through oligonucleotide base-pairing have been reported (for instance see Recombination induced by triple-helix-targeted DNA damage in mammalian cells, Faruqi AF, Seidman MM, Segal DJ, Carroll D, Glazer PM., Mol Cell Biol. 1996 Dec;16(12):6820-8; Correction of a splice-site mutation in the beta-globin gene stimulated by triplex-forming peptide nucleic acids; Chin JY, Kuan JY, Lonkar PS, Krause DS, Seidman MM, Peterson KR, Nielsen PE, Kole R, Glazer PM.; Proc Natl Acad Sci U S A. 2008 Sep 9;105(36):13514-9. Epub 2008 Aug 29. Preferably, cleavage of the sequence of interest is obtained using a double-strand break-inducing endonuclease. Nickases are generally less efficient than double-strand break-inducing endonucleases but may be safer in some instances (see Metzger et al., 2011, Nucl Acids Res 39(3): 326-35.)

In a preferred embodiment, the methods of the present invention are implemented as follows:
- a double-strand break is induced in the genome of the target cell with an endonuclease specific for the dystrophin gene sequence of interest as defined above; and
- the nucleic acid construct as defined above is provided as a template for homologous recombination at the double-strand break site.

As mentioned above, in a particular embodiment, the double-strand break-inducing endonuclease is specific of an intron of dystrophin, more particularly of intron 44.

Several means are known for achieving genome surgery. For example, one can use endonucleases such as zinc finger nucleases (ZFNs), meganucleases (MNs), and transcription activator-like effector nucleases (TALENs) to cut at specific site in the DNA. Endonucleases useful for the invention and methods for their engineering are well-known in the art.

In any of the methods described herein, the nuclease can be, for example, one or more zinc finger nucleases, one or more homing endonucleases (meganucleases) and/or one or more TAL-effector domain nucleases ("TALEN"). The nucleases (e.g., ZFN, Homing meganuclease and/or TALEN) as described herein may bind to and/or cleave the region of interest in a coding or non-coding region within or adjacent to the gene, such as, for example, a leader sequence, trailer sequence or intron, or within a non-transcribed region, either upstream or downstream of the coding region.

Zinc-finger nucleases (ZFNs) are artificial restriction enzymes generated by fusing a zinc finger DNA-binding domain to a DNA-cleavage (the Fokl nuclease) domain. The zinc finger domains can be engineered to target desired DNA sequences, which then enables the nuclease domain to cleave unique sequences within a complex genome. By taking advantage of the endogenous DNA double-strand break repair machinery, ZFN reagents can be used to precisely alter the genomes of higher organisms. The most straightforward method to generate new zinc-finger arrays is to combine smaller zinc-finger "modules" of known specificity. The most common modular assembly process involves combining three separate zinc fingers that can each recognize a 3 basepair DNA sequence to generate a 3-finger array that can recognize a 9 base-pair target site. Other procedures can utilize either 1 -finger or 2-finger modules to generate zinc-finger arrays with six or more individual zinc fingers. Methods of engineering zinc finger domains are known, and the ZFNs are available from a commercial supplier or can be developed through publically available reagents.

Several groups are also developing other types of engineered nucleases including engineered homing endonucleases, such as meganucleases (Grizot et al., 2009, Nucl. Acids Res., 37:5405-5419; Gao et al, 2010 Plant J., 61: 176-187; Smith et al., 2006, Nucl. Acids Res. 34: e149; Grizot et al., 2011, Nucl. Acids Res. 39: 6124-6136; Daboussi et al., 2012, Nucl. Acids Res. epub March 29, 2012) and nucleases based on engineered transcription activator like (TAL) effectors (Christian et al., 2010, Genetics, 186:757-761; Li et al, 2010, Nucl. Acids Res., 39:359-372). TAL effector nucleases (TALENs) are particularly interesting because TAL effectors appear to be very simple to engineer (Moscou et al, 2009, Science 326: 1501; Boch et al, 2009, Science 326: 1509-1512).

In a particular embodiment, a meganuclease is used, derived from I-CreI and engineered as described previously (Smith et al., 2006, Nucl. Acids Res. 34: e149; Grizot et al., 2011, Nucl. Acids Res. 39: 6124-6136; Daboussi et al., 2012, Nucl. Acids Res. epub March 29, 2012). In particular, meganucleases have been designed and developed to target intron 44 within the DMD gene upstream of a mutation hotspot. These meganucleases have previously been used as reported in Rousseau et al., 2011, J Gene Med, 13: 522-537 (named MGN3631 and MGN3633 in this article). Synthesis and validation of the meganucleases is as described in Daboussi et al., 2012 (previously cited). Results from next generation sequencing suggest that MGN3631 and MGN3633 cleavage point within its target site is between residues 9 and 10 of the following sequence: AATGTCTGATGTTCAATGTGTTGA (SEQ ID NO:1). The meganuclease is to be used along with a nucleic acid construct intended to repair the mutations resulting from mutations in this hotspot, for example for repairing deletion of exons 45-52.

The endonuclease implemented in the invention may be introduced in the target cell by methods known in the art. A gene coding for a nuclease may for example be delivered by viral or non-viral methods. In addition, means for delivering a nuclease protein are also known. As an illustrative, non-limiting, example one can cite the use of viral vectors carrying an endonuclease expression cassette. This expression cassette contains all the elements necessary for expression of the endonuclease gene, such as a promoter, termination sequences and enhancer sequences. The promoter may be ubiquitous or tissue-specific. In addition, the endonuclease expression cassette may be introduced in an integration-deficient or integration-competent virus, such as a lentivirus. Integration-deficient lentiviruses are preferably used. Methods for the production of such vectors are known in the art (e.g. in Jacome et al., 2009, Mol Ther 17: 1083-1092).

The nucleases, in particular DSB-inducing endonucleases, described above can be used to rewrite the sequence of an allele by invoking the homologous recombination machinery to repair the break, in particular double-strand break, using the supplied nucleic acid construct as a template. The homologous recombination machinery searches for homology between the damaged chromosome and the extra-chromosomal fragment (here, a cDNA block of dystrophin) and copies the sequence of the fragment between the two broken ends of the chromosome, regardless of whether the fragment contains the original sequence.

The above disclosure generally describes the present disclosure, which is further described by the following examples. These specific examples are provided solely for purposes of illustration, and are not intended to limit the scope of this disclosure. Although specific targets, terms, and values have been employed herein, such targets, terms, and values will likewise be understood as exemplary and non-limiting to the scope of this disclosure.

### EXAMPLES

### Materials and methods

### Cells

Immortalised human DMD myoblasts, carrying a deletion of exons 45-52, have kindly been made available to us through collaboration with the AFM-IdM (Paris). These cells, together with primary human skeletal muscle cells (hSkMCs) (TCS Cellworks, Buckingham, UK), 293T cells, and a second human DMD cell line carrying a deletion of exons 48-50 (from AFM-IdM, Paris) were cultured according to published protocols (Popplewell et al., 2011, Methods Mol Biol 709:153-78; Popplewell et al., 2010, Neuromuscul Disord 20:102-10), and used in experiments as indicated.

### Construction of LV-based MN and targeting matrix plasmids

A meganuclease (MN-DMD31), derived from I-CreI and engineered as described previously (Smith et al., 2006, Nucl. Acids Res. 34: e149; Grizot et al., 2011, Nucl. Acids Res. 39: 6124-6136; Rousseau et al., 2011, J Gene Med, 13: 522-537; Daboussi et al., 2012, Nucl. Acids Res. epub March 29, 2012), has been designed and developed to target different intron 44 within the DMD gene, upstream of a mutation hotspot. The MN-DMD31 construct was subcloned into a MluI/SwaI-digested lentiviral transfer plasmids (pRRLscSeGFP- CMV-WPRE) containing two expression cassettes. The first cassette encodes eGFP under the control of an internal spleen focus forming virus (SFFV) promoter, while the second contains a cytomegalovirus promoter and MN-DMD31. It also contains an SV40 polyadenylation signal and woodchuck hepatitis virus (WHP) posttranscriptional regulatory element (WPRE) sequence, flanking the late reverse transcriptase.

The targeting matrix was designed to contain a cDNA block encoding exons 45 to 52, flanked arms of homology to intron 44, synthetic splicing donor and acceptor, and intronic splicing enhancer sequence from rat FGFR2 gene (DICE element). The different elements within the targeting matrix were designed to be flanked by unique restriction enzyme sites to allow the modification of the matrix as required. The flanking arms were completely homologous to intron 44 either side of the DSB produced by MN-DMD31 cleavage. The degree of polymorphism in the 3kb sequence either side of the DSB was elucidated by PCR amplification of harvested genomic DNA from the four cell types (DMD del 45-52, DMD del 48-50, normal hSkMCs, 293T) with overlapping primers. Genomic DNA was harvested using DNeasy blood and tissue kit (Qiagen, Crawley, UK), and PCR performed using 2 x PCR Mastermix (GeneSys, Camberley, UK). The PCR products were sequenced (GATC- Biotech, Switzerland), and the sequences aligned to show position of polymorphic residues using VectorNTI software (Invitrogen, Paisley, UK). A targeting matrix was synthesized (by GeneART, Germany) with arms homologous to DMD del 45-52. The matrix (S2) had 1.5kb arms of homology. The synthesized targeting matrix was then inserted invertedly into a lentivector backbone (pRRLsinPPT-ISce-IT). Establishment of correction synthesis and sub-cloning was confirmed by restriction enzyme digests used according to manufacturers' instructions (New England Biolabs, Herts., UK).

### LV vector production and titration

For the expression of targeting matrix, integration-deficient lentiviral vectors, pseudotyped with the VSV-G envelope glycoprotein were used, while integration-competent lentiviral vectors were used for the expression of the meganuclease; these were produced as previously described (Jacome et al., 2009, Mol Ther 17:1083-1092). Briefly, lentiviral vectors were generated by transient transfection of HEK293T cells with the pMD2.VSV-G, pRSV.REV and pMDLg/pRREintD64V packaging plasmids and the transfer plasmid. At 24hrs after transfection, the harvested HEK293T cell medium was centrifuged at 690 x g for 10 min at room temperature and then filtered through a 0.22 µm filter (Nalgene, Rochester, NY, USA) to remove cell debris. The filtered medium was then harvested and transferred to high speed polyallomer centrifuge tubes (Beckman, Brea, CA, USA) and centrifuged at 50, 000 x g in a SW32Ti rotor (Beckman) for 2h at 4 °C. The vector was then resuspended in Dulbecco's modified Eagle's medium (Invitrogen, Paisley, UK), centrifuged at 1400 x g for 10 min and incubated with 5U ml⁻¹ DNaseI (Promega, Madison, WI, USA) and 10 mM MgCl2 (Sigma, UK) for 30 min. The vector was then aliquoted and stored at -80 °C. For viral titration, HeLa cells were transduced with serial dilutions of vector stock in the presence of 8 µg/ml polybrene. *eGFP* expression controlled by SFFV promoter in LV-MN was evaluated by FACS analysis. eGFP+ cells were scored using FACS analysis 72hr after infection. Real-time PCR titration of total vector DNA by late reverse transcript amplicon quantification was also used. Briefly, DNA extracted from cells (DNeasy, Qiagen, Crawley, UK) 24hr after transduction was subjected to PCR amplification. The final number of LV vector molecules in the transduced cells was determined by quantitative-PCR (q-PCR), using Taqman detection of PCR products in real time with the MyiQ single-color detection system (Bio-Rad, Hercules, CA). qPCR values were normalised by quantifying copies of the gene encoding actin in the DNA extracts using SYBR green detection system and correcting for Hela cell aneuploidy.

### Western blot analysis of meganuclease expression

Total protein extraction from del45-52 myoblast cell lines 2 days and 5 days after transduction of ICLV expressing MN-DMD31 (3631 or 3633) was performed with RIPA (150 mM sodium chloride, 1% NP-40, 0.5% sodium deoxycholate, 0.1% SDS, 50 mM Tris pH 8.0) buffer. 10 µg of total protein extract was electrophoretically separated on 3-8% Tris-acetate gels (Invitrogen, Paisley, UK), and then transferred onto nitrocellulose (Invitrogen, Paisley, UK). The MN expression was revealed using a specific I-Cre-I mouse monoclonal antibody. Alpha-tubulin antibody (ab4074) (Abcam, Cambridge, UK) was used as a loading control.

### Assessment of MN activity by deep gene sequencing

To evaluate the efficiency of MN-DMD31 (two variants) to cut DNA at its target site, human DMD del 45-52 cell lines were transduced with ICLV-MN at MOIs of 1 and 0.5. Five days after transduction, genomic DNA was purified using DNeasy blood and tissue kit (Qiagen, Crawley, UK), a ∼400bp product amplified around the MN target site by PCR using 2 x PCR Mastermix (GeneSys, Camberley, UK). The purified products were deep gene sequenced (by GATC-Biotech, Switzerland) to assess the mutation rate as a result of non-homologous end joining (NHEJ) repair of the DSB induced by MN-DMD31 at its target site in intron 44 of the DMD gene, compared to non- treated controls. Standard PCR protocols within the knowledge of those skilled in the art were used.

Detection of corrected dystrophin genomic DNA in human DMD del 45-52 cell lines Human DMD del 45-52 cells were transduced in 24-well plates with 1000 MOI of ICLV-MN and IDLV-targeting matrix, as assessed by qPCR. After 72h, genomic DNA was harvested using DNeasy blood and tissue kit (Qiagen, Crawley, UK). Semi-nested PCR using LongAmp polymerase (New England Biolabs, Herts., UK) was performed to show homologous recombination had occurred between intron 44 in targeting matrix and endogenous intron 44. The products were separated on 1% agarose gels in Tris-borate/EDTA buffer and HyperLadder I (Bioline, London, UK) was used as marker. Standard PCR protocols within the knowledge of those skilled in the art were used.

### Detection of corrected dystrophin mRNA in human DMD del 45-52 cell lines

Human DMD del 45-52 cells were transduced in 24-well plates with 1000 MOI of ICLV-MN and IDLV-targeting matrix, as assessed by qPCR. After 72h, RNA was harvested using QIAshredder and RNeasy extraction kit (Qiagen, Crawley, UK) and nested RT-PCR performed to examine corrected mRNA expression using GeneScript RT-PCR system kit (GeneSys, Camberley, UK) for the first round, and 2 x PCR Mastermix (GeneSys, Camberley, UK) for the second round. The products were separated on 1.2% agarose gel in Tris-borate/EDTA buffer and Hyper Ladder IV (Bioline, London, UK) was used as marker.

### Primers

Primers used to assess homologous recombination between endogenous DNA and repair matrix cDNA:
Left hand side:
   First round: DMD31-for3 (SEQ ID NO:14) and KI h45 rev (SEQ ID NO:15)
   Second round: DMD31-for4 (SEQ ID NO:16) and KI h45 rev
   Right hand side:
First round: h51f(SEQ ID NO:17) and DMD31-Rev12 (SEQ ID NO:18)
   Second round:h51f and DMD31-Rev11 (SEQ ID NO:19)
   Primers used to assess splicing of repair matrix cDNA into endogenous mRNA:
   Left hand side:
First round: h43f (SEQ ID NO:20) and h48r (SEQ ID NO:21)
   Second round: h44f (SEQ ID NO:22) and h46r (SEQ ID NO:23)
   Right hand side:
   First round: h50f(SEQ ID NO:24) and h55r (SEQ ID NO:25)
   Second round: h51f and h54r (SEQ ID NO:26)

### Results

### 1. Building of targeting repair matrices

### 1.1.Elucidation of sequence required for arms of homology

The aim of the endonuclease-mediated gene correction is to insert a cDNA block of missing exons with flanking splice sites, or other regulatory sequence elements, into the endonuclease-induced DSB within the intron harbouring the deletion junction. An endonuclease has been designed and developed to target intron 44 within the DMD gene, upstream of a mutation hotspot, and packaged into a lentivirus vector to allow contemporaneous and transient endonuclease expression. Arms of homology are required to drive homologous recombination between genomic DNA and repair matrix at endonuclease-induced DSB site, and subsequent cDNA knock-in. To establish complete homology and elucidate positions of any polymorphic residues, sequencing of the intron 44 (6 kb) surrounding the endonuclease target site was performed on PCR amplicons produced using overlapping primers on genomic DNA from 293T cells, del 45-52 DMD cells and del 48-50 DMD cells. Alignment of the 6861 bp sequences from the three different genomic DNAs is shown in Fig. 1.

### 1.2 Incorporation of repair matrix elements

The cDNA block, flanking splice sites and arms of homology have been incorporated into a repair matrix (Fig. 2). A basic targeting construct, with 1.5kb arms of homology (S2), with cDNA encoding the block of exons 45 to 52, which are missing in del45-52 human DMD cells, was synthesized commercially, and artificial flanking splice sites (synthetic branch point, polypyrimidine tract and splice acceptor site, synthetic splice donor and intronic splicing enhancer sequence from rat FGFR2 gene (DISE element) incorporated. The synthesized targeting matrix was then inserted invertedly into an integration-deficient lentivector (IDLV) backbone to avoid aberrant and unexpected splicing of the mRNA viral genome. Establishment of correction synthesis and sub-cloning was confirmed by restriction enzyme digestion (data not shown). IDLV vectors were generated by standard transient transfection technology in 293T cells. Real-time PCR was used to accurately quantify viral genomes (data not shown). Titres of 5x10e8 were typically achieved.

### 2. Assessment of repair matrix function

### 2.1. Ability of repair matrix to drive homologous recombination

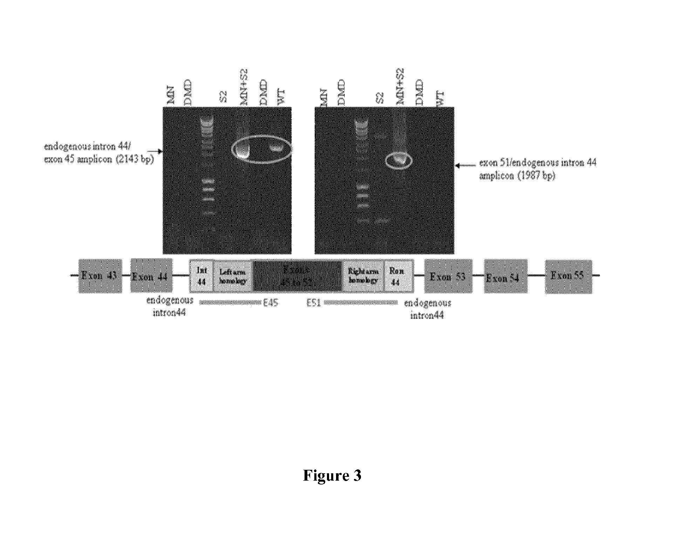

To evaluate the potential of endonuclease and targeting matrix to induce gene repair by homologous recombination and to elucidate repair matrix function and efficacy, del45-52 human DMD cells were infected with 1000 of MOI of LV-endonuclease and IDLV-targeting matrix (S2) (as quantified by real-time PCR). After 72h, genomic DNA was harvested and semi-nested PCR using LongAmp polymerase was performed using second round primers to amplify from endogenous intron 44 upstream of the 1.5 kb left arm of homology to exon 45 (Fig. 3a) and from exon 51 to endogenous intron 44 downstream of the 1.5 kb right arm of homology (Fig. 3b). Highlighted products show that homologous recombination has occurred between endogenous intron 44 and the right and left arms of homology within the targeting matrix (S2), as a specific consequence of endonuclease cleavage.

### 2.2. Ability of repair matrix to drive cDNA knock-in

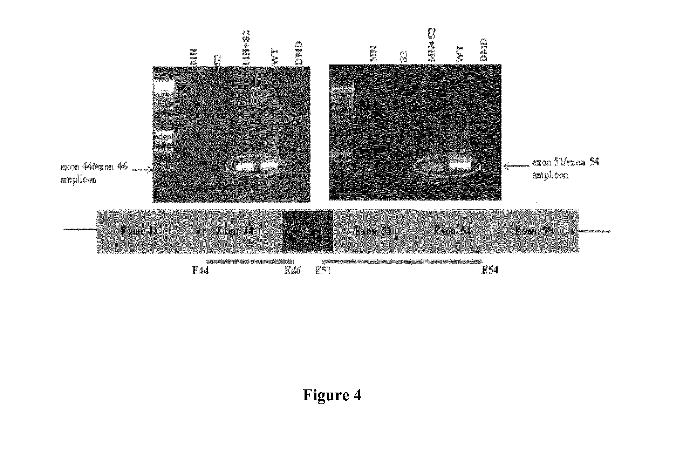

To evaluate the potential of endonuclease and targeting matrix to induce gene repair by homologous recombination and knock-in of exons 45 to 52, del45-52 human DMD cells were co-transduced in 24-well plates with 1000 of MOI of LV-endonuclease and IDLV-targeting matrix S2 (as quantified using real-time PCR). After 72h, RNA was harvested and nested RT-PCR performed to examine corrected mRNA expression. Second round primers were used to amplify from exon 44 (endogenous) to exon 46 (matrix) (Fig. 4a), and to amplify from exon 51 (matrix) to exon 54 (endogenous) (Fig. 4b). The highlighted products show that exon 45 to 52 cDNA from the targeting matrix has been successfully knocked-in between endogenous exons 44 and 53 and this corrected genomic DNA was successfully transcribed to mRNA.

### 2.3. Assessment of the efficiency of the synthetic splice machinery to drive splicing

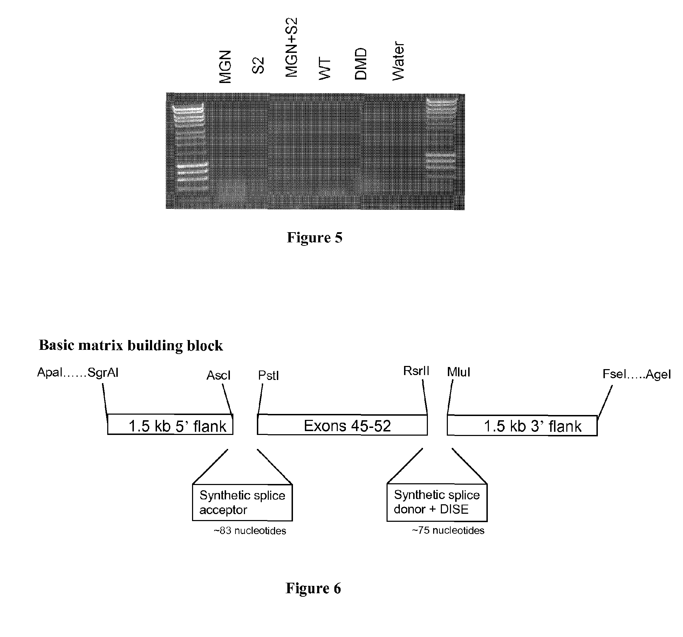

To evaluate the efficiency of the synthetic splicing machinery to drive splicing, del45-52 human DMD cells were transduced in 24-well plates with 1000 of MOI of LV-endonuclease (MGN) and IDLV-targeting matrix (S2). After 72h, RNA was harvested, DNase-treated and subjected to nested RT-PCR to show that splicing is efficiently occurring in the RNA between exon 52 in the targeting matrix and endogenous exon 53 (Fig. 5). The primers used were designed to bind to exon 51 in the targeting matrix and intron 44 in the right arm of homology, so that product would be generated only if the mRNA was not effectively spliced. The absence of product implies that the synthetic splice donor is efficiently driving splicing of exon 52 from the cDNA block within the repair matrix to endogenous exon 53. The repair matrix has been designed in such a way to allow modification of the splice elements if required.

### 3. Illustration of possible modifications in the repair matrix of the invention

Using in-depth sequence analysis and standard DNA synthesis technology, unique restriction sites were incorporated into the repair matrix to allow its stepwise modification as required as shown in figure 6.

Modifications are facilitated using the restrictions enzymes provided in the matrix.

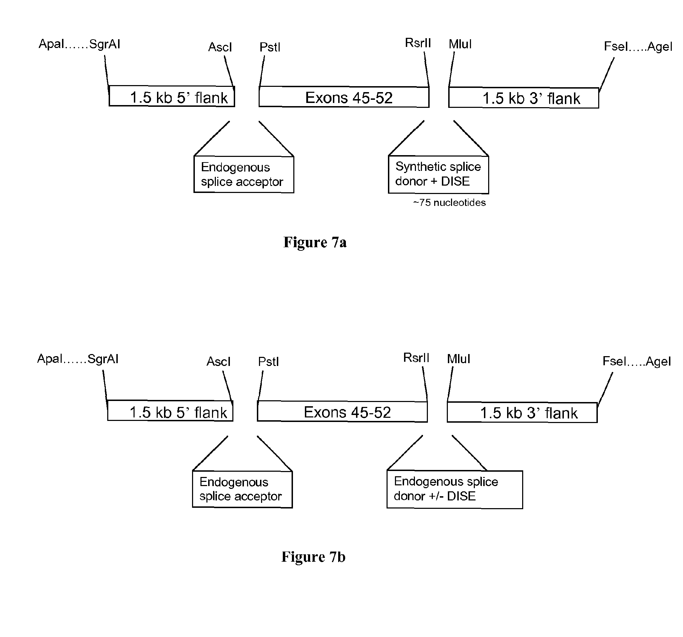

Figure 7a illustrates the replacement of the synthetic acceptor present in the matrix of figure 6 by an endogenous (or other) splice acceptor. This can be carried out by cutting the matrix with AscI and PstI restriction enzymes, thereby removing the synthetic splice acceptor, and then ligating in the endogenous acceptor.

Figure 7b illustrates the replacement of the synthetic splice donor present in the matrix of figure 7a by an endogenous (or other) splice donor. This can be carried out by cutting the matrix with RsrII and MluI restriction enzymes, thereby removing the synthetic splice donor, and then ligating in the endogenous donor.

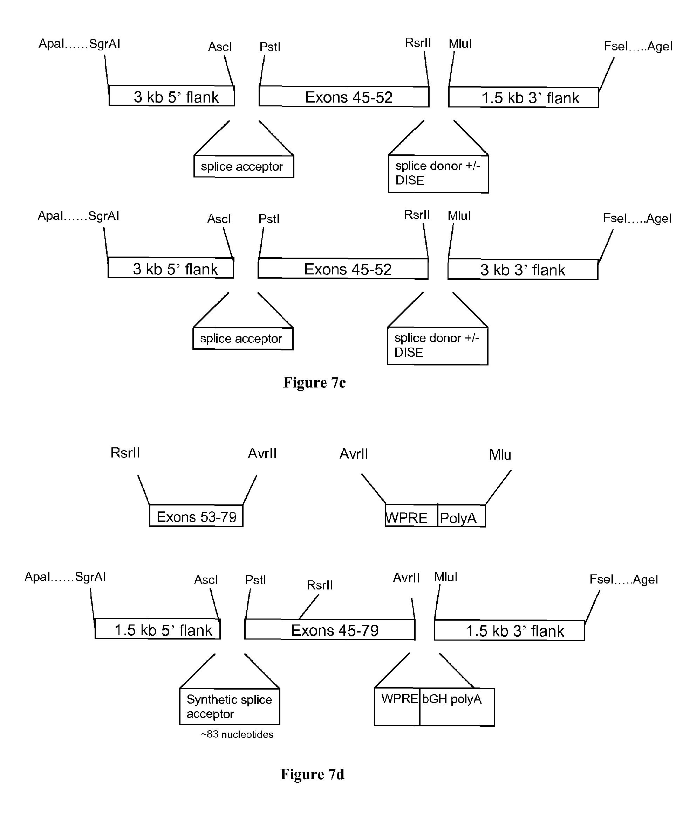

Figure 7c illustrates the modification of the length of the arms of homology.

To change the right arm of homology to 3 kb, the matrix is cut with AscI and ApaI (or SgrAI) and a PCR amplified 3 kb sequence from delta 45-52 genomic DNA with primers with AscI and ApaI (or SgrI) restriction sites incorporated is ligated in. Alternatively, the matrix is cut at unique restriction sites with AccI or FblI or XmiI and is ligated a 2+ kb sequence of intron (i.e. 2262 bp) obtained by PCR amplification from delta 45-52 genomic DNA with primers incorporating AccI (or FblI or XmiI) and ApaI (or SgrAI).

To change the left arm of homology to 3 kb, the matrix is cut with MluI and AgeI (or FseI) and a PCR amplified 3 kb sequence from delta 45-52 genomic DNA with primers with MluI and AgeI (or FseI) restriction sites incorporated is ligated in. Alternatively, the matrix is cut at unique restriction sites with BanIII or Bsa29I or BspXI or with BarI and is ligated a 2+ kb sequence of intron (i.e. 2098 or 2088 bp) obtained by PCR amplification from delta 45-52 genomic DNA with primers incorporating BanIII (or other) and AgeI (or FseI).

Figure 7d illustrates the replacement of the cDNA block with a block of exons 45 to 79 and 1 kb arms of homology. In the present example, due to presence of three PstI sites in extended 3 kb arms, all modifications must be done prior to putting in the longer arms. To change the exon 45-52 block to exon 45-79, and splice donor + DISE to WPRE/bGH polyA, the basic matrix of figure 7a is cut with RsrII and MluI and exon 53-79 cDNA block amplified with primers with incorporated RsrII and AvrII, and WPRE/bGHpolyA flanked with AvrII and MluI restriction sites are ligated into the cut matrix.

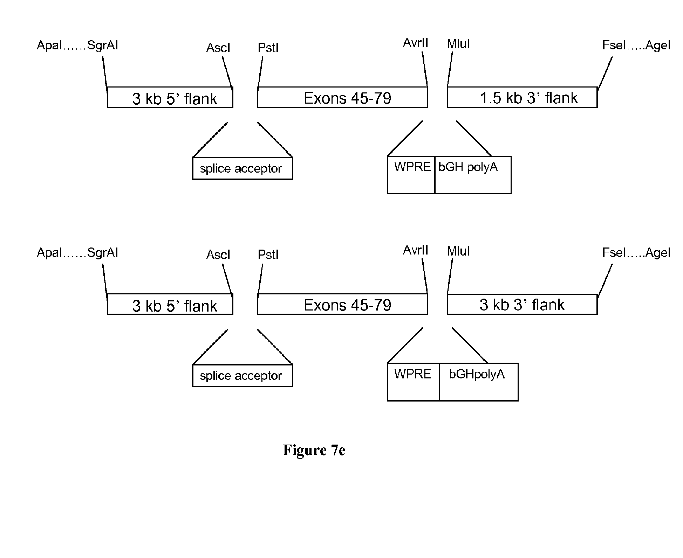

Figure 7e illustrates the change of the length of the arms of homology to 3 kb within the matrix of figure 7d comprising a cDNA block encoding exons 45 to 79.

To change the right arm of homology to 3 kb, the matrix is cut with AscI and ApaI (or SgrAI) and ligated with a PCR amplified 3 kb sequence from delta 45-52 genomic DNA with primers with AscI and ApaI (or SgrA1) restriction sites incorporated. Alternatively, the matrix is cut at a unique restriction site with AccI or FblI or XmiI and ligated with a 2+ kb of PCR amplified fragment of an intron (i.e. 2262 bp) from delta 45-52 genomic DNA obtained with primers incorporating AccI (or FblI or XmiI) and ApaI (or SgrAI).

To change the left arm of homology to 3 kb, the 5' modified matrix is cut with MluI and AgeI (or FseI) and ligated with a PCR amplified 3 kb sequence from delta 45-52 genomic DNA with primers with MluI and AgeI (or FseI) restriction sites incorporated. Alternatively, the matrix is cut at a unique restriction site with BanIII or Bsa29I or BseCI or BspXI (position 902) or with BarI and ligated with a 2+ kb of PCR amplified fragment of an intron (i.e. 2098 or 2088 bp) from delta 45-52 genomic DNA obtained with primers incorporating BanIII (or one of the other restriction site) and AgeI (or FseI).

## Claims

1. A nucleic acid construct comprising a first and second portions which are homologous to regions 5' and 3' of a dystrophin gene sequence of interest, further comprising a third portion positioned between the first and second portion, said third portion comprising a dystrophin correcting cDNA flanked either sides by appropriate regulatory sequences.

2. The construct according to claim 1, wherein the first and second portions are homologous to a region of interest comprised in intron 44 of the dystrophin gene, and the third portion corresponds to a cDNA comprising exons downstream of intron 44 in the dystrophin gene.

3. The construct according to claim 2, wherein said cDNA comprises exons 45 to 79 or exons 45 to 52 of the dystrophin gene.

4. The construct according to any one of claims 1 to 3, wherein a splice acceptor is present between the first and third portions and a splice donor, optionally flanked by an intronic splicing enhancer sequence, is present between the third and second portions.

5. The construct according to any one of claims 1 to 3, wherein a splice acceptor is present between the first and third portions and a WPRE and polyA sequence is present between the third and second portions.

6. The construct according to any one of claims 1 to 5, wherein the first and second portions are independently between 0.5 kb and 8 kb in length.

7. The construct according to any one of claims 1 to 6, wherein a first unique restriction site is present at the start of the third portion and/or a different second unique restriction site is present at the end of the third portion.

8. The construct according to claim 7, wherein the third portion corresponds to exons 45 to 52 of the dystrophin gene and the first restriction site is a PstI site and the second restriction site is a RsrII site.

9. The construct according to any one of claims 1 to 6, wherein the first portion comprises the nucleic acid sequence shown in SEQ ID NO:2 or SEQ ID NO:3 and the second portion comprises the nucleic acid sequence shown in SEQ ID NO:4 or SEQ ID NO:5.

10. A recombinant plasmid comprising the nucleic acid construct according to any one of claims 1 to 9.

11. A recombinant viral vector comprising incorporated into its genome a nucleic acid construct according to any one of claims 1 to 9.

12. The viral vector according to claim 11, being a lentiviral vector.

13. A nucleic acid construct according to any one of claims 1 to 9, the plasmid of claim 10 or the viral vector of claim 11 or 12, for use in a method of treatment of a disease caused by a mutation in the dystrophin gene, in particular for the treatment of Duchenne muscular dystrophy or Becker muscular dystrophy.

14. A nucleic acid construct according to any one of claims 1 to 9, the plasmid of claim 10 or the viral vector of claim 11 or 12, for use in a method for restoring full-length dystrophin expression in a subject in need thereof, for altering the genomic DNA sequence of the dystrophin gene in a target cell, for repairing the dystrophin gene in the genome of a target cell, or for inserting a cDNA block of missing exons in the dystrophin gene in the genome of a target cell.

15. The nucleic acid construct, the plasmid or the viral vector according to any one of claims 13 to 14, wherein the method comprises cleaving the dystrophin gene in the genome at a target sequence of interest, in particular in intron 44, and providing said nucleic acid construct as a template for homologous recombination at the double-stand break site.

16. The nucleic acid construct, the plasmid or the viral vector according to claim 15, wherein the cleavage correspond to a double strand-break induced with an nuclease specific for the target sequence of interest in the dystrophin gene.

17. The nucleic acid construct, the plasmid or the viral vector according to claim 16, wherein at least one nuclease is a zinc finger nuclease, a TALEN or a homing endonuclease or other entities able to induce matrix-mediated homologous recombination.
